# EUROPÄISCHE PATENTANMELDUNG

(11) **EP 3 861 967 A1**
(43) Veröffentlichungstag der Anmeldung: **11.08.2021**
(21) Anmeldenummer: 21155882.0
(22) Anmeldetag: 08.02.2021
(51) Int. Cl.: A61F 5/02, A41D 1/00, A61F 5/01, A61F 5/24, A61F 5/26, A61F 5/28, A41B 1/08

(54) **THERAPEUTISCHES OBERBEKLEIDUNGSSTÜCK, INSBESONDERE TEXTILES RUMPFKORSETT, UND VERFAHREN ZU DESSEN ANPASSUNG AN EINEN ZU BEHANDELNDEN TRÄGER**

(30) Priorität: 07.02.2020 DE 202020100653 U
(71) Anmelder: FXF GmbH, 92224 Amberg (DE)
(72) Erfinder: FISCHER, Franz, 92224 Amberg (DE)
(74) Vertreter: Benninger, Johannes

(57) **Zusammenfassung**

Die Erfindung betrifft ein Oberbekleidungsstück (22) zur äußeren Einwirkung auf einen Oberkörper eines Trägers oder Patienten (10) mittels Stütz- und/oder Zugkräften, insbesondere zur Abstützung und/oder zur therapeutischen Behandlung von Wirbelsäulenstellungen, die von einer aufrechten Normalstellung abweichen, etwa im Zusammenhang mit dem Krankheitsbild einer Skoliose.

Zwischen mindestens zwei voneinander entfernten Abschnitten (24, 28) des Oberbekleidungsstückes (22) befindet sich mindestens ein Zugelement (32, 34), das die mindestens zwei voneinander beabstandete Abschnitte (24, 28) verbindet und Zugkräfte zwischen den mindestens zwei Abschnitten (24, 28) erzeugt.

Das mindestens eine Zugelement (32, 34) verfügt über elastische Eigenschaften, die sich von den elastischen Eigenschaften des Oberbekleidungsstückes (22) dahingehend unterscheiden, dass die elastischen Rückstellkräfte des mindestens einen Zugelementes (32, 34) stärker sind als diejenigen Bereiche des Oberbekleidungsstückes (22), die sich zwischen den mindestens zwei Abschnitten (24, 28) befinden.

Die Erfindung betrifft weiterhin ein Verfahren zur Anpassung und/oder Variation von auf einen Oberkörper eines Trägers oder Patienten (10) einwirkenden Stütz- und/oder Zugkräften mittels eines Oberbekleidungsstückes (22), welches insbesondere der Abstützung und/oder zur therapeutischen Behandlung von Wirbelsäulenstellungen, die von einer aufrechten Normalstellung abweichen, dienen kann. Die Anpassung erfolgt durch Umsetzen und/oder Austausch von einzelnen Zugelementen (32, 34) am Oberbekleidungsstück.

## Beschreibung

Die vorliegende Erfindung betrifft ein therapeutisches Oberbekleidungsstück, insbesondere ein textiles Rumpfkorsett zur Abstützung und zur therapeutischen Behandlung von Wirbelsäulenstellungen mit den Merkmalen des unabhängigen Anspruchs 1. Die Erfindung betrifft zudem ein Verfahren zur Anpassung und/oder Variation eines solchen Oberbekleidungsstückes an einen zu behandelnden Träger mit den Merkmalen des unabhängigen Verfahrensanspruchs.

Beim Krankheitsbild der vereinzelt beim Menschen auftretenden sog. Skoliose handelt es sich um seitliche Verkrümmungen mit überlagerter Verdrehung der Wirbelsäule, was in aller Regel von strukturellen Verformungen einzelner oder mehrerer Wirbelkörper begleitet ist. Da bei einer Skoliose die Muskulatur typischerweise nicht in der Lage ist, den Lageabweichungen der Wirbelsäule entgegenzuwirken, verläuft diese ohne stützende Maßnahmen in mehreren Bögen, die zur Aufrechterhaltung des körperlichen Gleichgewichts des aufrecht stehenden Betroffenen normalerweise gegenläufig und in unterschiedlicher S-Form verlaufen können.

Als Therapie für eine Skoliose kommen einerseits eine klassische Physiotherapie, meist aber eine Korsettbehandlung, aber auch versteifende Wirbelsäulenoperationen in Frage, wobei diese Therapien häufig miteinander kombiniert werden. Die deutlich am häufigsten angewandten Therapievarianten bestehen in einer kombinierten Physiotherapie und in einer Abstützung des Oberkörpers mittels Korsett oder Orthese. Demgegenüber sind versteifende Operationen an der Wirbelsäule deutlich seltener notwendig.

Ein Korsett bzw. eine sog. Orthese dienen hierbei nicht nur zur Abstützung, sondern auch zur Lenkung des Wachstums bei jugendlichen Patienten, mit dem Ziel, eine Verschlechterung des Zustandes zumindest aufzuhalten, im Idealfall jedoch eine Verbesserung zu erreichen, indem stärkere Abweichungen vom Normalzustand korrigiert werden. Zum Einsatz kommen häufig sog. Chêneau-Korsetts, die den gesamten Rumpf des Patienten umschließen, aber durch ihren asymmetrischen Aufbau der Verdrehung der Wirbelsäule ebenso entgegenwirken können wie einer seitlichen Verkrümmung. Ein solches Chêneau-Korsett wird üblicherweise mittels Gipsabdruckes hergestellt, wonach ein Orthopädietechniker nicht nur das Korsett anfertigt, sondern auch Druckpolster positioniert, sog. Pelotten.

Nicht zuletzt die Größenausdehnung herkömmlicher Chêneau-Korsetts bedingt einen vergleichsweise aufwendigen Herstellungsprozess, da der für die Fertigung ausgebildete Orthopädietechniker nach Abnahme einer für jeden Patienten individuellen Negativform aus Gips die beiden Halbschalen des Korsetts, die in aller Regel an aneinanderstoßenden Längskanten durch Scharnierverbindungen aneinander hängen und an den gegenüber liegenden Längskanten nach dem Anpassen an den Patienten lösbar verbunden werden. Zuvor werden an definierten Punkten in den Halbschalen die ebenfalls jeweils individuell anzupassenden Druckpolster eingesetzt, die vorzugsweise je nach Heilungsverlauf und Haltungsveränderung des Patienten über den Zeitverlauf in ihren Positionen verändert und/oder durch größere oder kleinere Druckpolster ausgetauscht werden können.

Angesichts des Fertigungsaufwandes für herkömmliche Chêneau-Korsetts kann es als vorrangiges Ziel der vorliegenden Erfindung betrachtet werden, einfacher und kostengünstiger zu fertigende Maßnahmen oder Korsetts zur Behandlung des Krankheitsbildes der Skoliose zur Verfügung zu stellen, die zumindest denselben Therapieeffekt wie die bisher verwendeten Korsetts bieten können, die aber im Idealfall aufgrund einer leichteren individuellen Anpassbarkeit an den betroffenen Patienten sogar einen rascheren Therapieerfolg ermöglichen sollen. Angesichts des mangelhaften Trage- und Handhabungskomforts der herkömmlichen Chêneau-Korsetts ist zudem als weiteres Ziel der vorliegenden Erfindung die Verbesserung der Akzeptanz durch den Patienten zu nennen.

Darüber hinaus kann es als weiteres Ziel der Erfindung betrachtet werden, ein verbessertes und vereinfachtes Verfahren zur Anpassung solcher therapeutischen Einrichtungen oder Korsetts zur Verfügung zu stellen, das es erlaubt, sich verändernde Krankheitsbilder zu berücksichtigen, und das es erlaubt, die therapeutischen Maßnahmen bedarfsweise schnell und einfach modifizieren zu können.

Zumindest ein Teil der hier identifizierten Ziele der Erfindung werden durch den Gegenstand des unabhängigen Anspruchs 1 erreicht. Merkmale vorteilhafter Weiterbildungen der Erfindung ergeben sich aus den jeweiligen abhängigen Ansprüchen. Nicht zuletzt verlangt es der unzureichende Trage- und Handhabungskomforts der herkömmlichen Chêneau-Korsetts, die Akzeptanz des erfindungsgemäßen textilen Korsetts durch den Patienten zu verbessern, was in erster Linie durch Vermeidung von steifen Schalenkörpern, Halbschalen oder sonstigen unnachgiebigen Korsett-Elementen erreicht werden soll.

So schlägt die Erfindung zur Erreichung der genannten Ziele ein neuartiges und sich in einigen wesentlichen Aspekten von den oben beschriebenen, seit längerer Zeit bekannten und vielfach therapeutisch eingesetzten Chêneau-Korsetts unterscheidendes textiles Oberbekleidungsstück vor, das als Rumpfkorsett dienen oder wirksam sein kann. Das textile Oberbekleidungsstück oder textile Rumpfkorsett weist mehrere Abschnitte, Wirkflächen und/oder Wirkbereiche auf, die untereinander mit nachfolgend näher beschriebenen Zugelementen, d.h. mit flexiblen und/oder elastischen Abschnitten verbunden sind. Das erfindungsgemäße Oberbekleidungsstück dient auf diese Weise der variabel und individuell einstellbaren Stabilisierung und Abstützung und damit insbesondere der therapeutischen Behandlung von Wirbelsäulenstellungen, die von einer aufrechten Normalstellung abweichen, wobei mit diesen Fehlstellungen insbesondere Krankheitsbilder gemeint sind, die im Zusammenhang mit einer Skoliose stehen.

Die Wirkungsweise des erfindungsgemäßen Oberbekleidungsstückes beruht auf dem Prinzip einer äußeren Einwirkung auf einen Oberkörper eines Trägers mittels Stütz- und/oder Zugkräften, insbesondere zur Abstützung und/oder zur therapeutischen Behandlung von Wirbelsäulenstellungen, die von einer aufrechten Normalstellung abweichen, wie sie insbesondere beim Krankheitsbild der Skoliose auftreten.

Zwischen mindestens zwei voneinander entfernten Abschnitten des Oberbekleidungsstückes befindet sich jeweils mindestens ein Zugelement, das die mindestens zwei voneinander beabstandete Abschnitte verbindet und Zugkräfte zwischen den mindestens zwei Abschnitten erzeugt.

Es ist vorgesehen, dass das mindestens eine Zugelement über elastische Eigenschaften verfügt, die sich von den elastischen Eigenschaften des Oberbekleidungsstückes dahingehend unterscheiden, dass die elastischen Rückstellkräfte des mindestens einen Zugelementes stärker sind als diejenigen Bereiche des Oberbekleidungsstückes, die sich zwischen den mindestens zwei Abschnitten befinden.

Vorzugsweise ist das mindestens eine Zugelement durch ein elastisches Band gebildet, dessen beide Enden jeweils an den voneinander beabstandeten Abschnitten verankert sind.

Da das Zugelement lösbar an mindestens einem Abschnitt verankert ist, können durch Auswahl eines geeigneten Zugelementes einer bestimmten Länge, einer bestimmten Stärke sowie mit definierten elastischen Rückstellkräften bestimmte Kräfte zwischen den voneinander entfernten Abschnitten des Oberbekleidungsstückes erzeugt werden, an denen die beiden Enden des Zugelementes jeweils verankert sind.

Vorzugsweise befinden sich an der Außenseite des erfindungsgemäßen Oberbekleidungsstückes mehrere solcher Abschnitte, die sinnvollerweise voneinander entfernt bzw. zumindest gering zueinander beabstandet sind.

Eine Variante des erfindungsgemäßen Oberbekleidungsstückes kann vorsehen, dass die mehreren an der Außenseite vorgesehenen Abschnitte jeweils gruppiert sind, wobei diese Gruppen mit jeweils mehreren voneinander beabstanden Abschnitten jeweils größere Bereiche bilden.

Diese Bereiche mit den mehreren Abschnitten können bspw. als streifenartige Areale ausgebildet sein, die sich jeweils entlang einer vertikalen Richtung der getragenen Oberbekleidung erstrecken. So sind Varianten sinnvoll, bei denen sich sowohl an der Vorderseite als auch an der Rückseite des Oberbekleidungsstückes mindestens jeweils zwei streifenartige Areale befinden, wobei diese streifenartigen Areale entweder parallel und in vertikaler Richtung verlaufen oder bspw. in Richtung von unten nach oben leicht V-förmig auseinanderstreben. Auf diese Weise können zwischen den einzelnen Bereichen dieser Areale mehrere Zugelemente verankert werden, wodurch sich die gewünschten Zugkräfte erzeugen lassen, die den Träger in der gewünschten Weise beeinflussen und ihn eine der Skoliose entgegenwirkende verbesserte Körperhaltung einnehmen lassen.

So kann eine bevorzugte Variante des erfindungsgemäßen Oberbekleidungsstückes vorsehen, dass sich die Bereiche oder streifenartigen Areale rückseitig und/oder vorderseitig jeweils entlang der linken und rechten Seite des Oberbekleidungsstückes erstrecken, und zwar von dessen unterer Kante (Hüftbereich) bis zum Schulterbereich (ggf. bis zum oberen Ausschnitt).

Um die Zugelemente leicht aufsetzen und auch wieder von den definierten Abschnitten oder Bereichen abziehen zu können, kann das Oberbekleidungsstück mit Klettflächen o. dgl. ausgestattet sein. Hierbei können die Abschnitte jeweils als erste Gegenflächen eines Verschlussmittels, insbesondere eines Klettverschlusses ausgebildet sein, während die Zugmittel an ihren endseitigen Abschnitten jeweils mit zweiten Gegenflächen des Verschlussmittels ausgestattet sind, so dass sich leicht lösbare, aber nach Herstellung der Haftverbindung sehr stabile Verankerungen der Zugelemente am Oberbekleidungsstück herstellen lassen.

Bei dieser Variante des Oberbekleidungsstückes können die Zugmittel kontaktierend auf der Oberfläche der Oberbekleidung verlaufen, sind jedoch nicht mit/in dieser integriert.

Bei einer alternativen Variante können die Zugmittel jedoch auch abschnittsweise oder komplett in die Oberbekleidung eingearbeitet sein, bspw. durch eine geeignete Integration der elastischen Zugmittel in das Gewebe oder Gestricke der Oberbekleidung. Eine solche Variante kann jedoch nach Applikation der Zugelemente nicht mehr wesentlich verändert werden, so dass eine individuelle Anpassung an den Träger und eine Neuanfertigung nach Veränderung seiner Körperhaltung und/oder seines Krankheitsbildes erforderlich ist.

Zusammenfassend schlägt die Erfindung zur Erreichung der oben genannten Ziele ein neuartiges und sich in einigen wesentlichen Aspekten von den oben beschriebenen, seit längerer Zeit bekannten und vielfach therapeutisch eingesetzten Chêneau-Korsetts unterscheidendes textiles Rumpfkorsett vor, das mehrere Wirkflächen und/oder Wirkbereiche aufweist, die untereinander mit flexiblen und/oder elastischen Abschnitten verbunden sind, und das der Stabilisierung und Abstützung und damit insbesondere der therapeutischen Behandlung von Wirbelsäulenstellungen, die von einer aufrechten Normalstellung abweichen, dient, wobei mit diesen Fehlstellungen insbesondere Krankheitsbilder gemeint sind, die im Zusammenhang mit einer Skoliose stehen.

Die mindestens zwei Wirkflächen und/oder Wirkbereiche, die oben auch ganz allgemein als mindestens zwei Abschnitte bezeichnet wurden, bilden bei dem erfindungsgemäßen textilen Oberbekleidungsstück oder Rumpfkorsett jeweils integrale Bestandteile dieses Oberbekleidungsstückes oder Korsetts. Wahlweise sind sie auch durch verstärkte textile Bereiche des Oberbekleidungsstückes oder des Rumpfkorsetts gebildet. Die wenigstens zwei Wirkflächen, die hier auch durch Wirkbereiche gebildet sein können, liegen jedenfalls flächig an definierten Rumpfbereichen des Patienten an und bilden gleichsam Ankerflächen für die Einleitung von korrigierenden Stützkräften, die im Wesentlichen durch die mit den Abschnitten, den Wirkflächen und/oder Wirkbereichen verbundenen flexiblen und/oder elastischen Abschnitten oder Zugelementen eingeleitet werden, indem diese flexiblen und/oder elastischen Abschnitte oder Zugelemente insbesondere an voneinander beabstandeten Wirkflächen und/oder Wirkbereichen verankert sind und zwischen diesen definierte Wechselwirkungen herstellen.

Die therapeutische Wirkung des erfindungsgemäßen Oberbekleidungsstückes oder Rumpfkorsetts beruht in erster Linie darauf, dass die mindestens zwei Wirkflächen und/oder Wirkbereiche mittels der flexiblen und/oder elastischen Abschnitte oder Zugelemente in einer Weise miteinander verbunden sind und damit in Wechselwirkung zueinander gebracht werden, dass die definierte Abschnitte des Rumpfbereichs des Patienten bedeckenden Wirkflächen und/oder Wirkbereiche in einen definierten Abstand zueinander und/oder im an den Patienten angelegten Zustand in eine definierte räumliche Zuordnung zueinander bringbar oder gebracht sind, wobei durch die elastischen Abschnitte oder Zugelemente eine definierte Zugspannung zwischen den Wirkflächen und/oder Wirkbereichen erzeugt ist.

Sinnvoll ist es außerdem, wenn die mindestens zwei voneinander entfernten Abschnitte, Wirkflächen und/oder Wirkbereiche, die mittels der flexiblen und/oder elastischen Abschnitte oder Zugelemente in definierten Abständen und unter definierter Zugspannung zueinander zusammengehalten sind, gemeinsam weniger als die Hälfte einer Gesamtoberfläche eines Rumpfbereichs eines Patienten zwischen dessen Beckenbereich und dessen Halsbereich bedecken, so dass die flexiblen und/oder elastischen Abschnitte jeweils ausreichende Krafteinleitungen zwischen den Wirkflächen oder Wirkbereichen gewährleisten können.

Bei dem erfindungsgemäßen Oberbekleidungsstück oder textilen Rumpfkorsett können die flexiblen und/oder elastischen Abschnitte oder Zugelemente insbesondere durch elastische Streifen gebildet sein, die höhere Rückstellkräfte und/oder höhere Spannkräfte ausbilden als das sie umgebende und/oder das an ihnen anliegende textile Material des Oberbekleidungsstückes oder Rumpfkorsetts.

In der Regel dürfte für das Material des Oberbekleidungsstückes oder Rumpfkorsetts ein geeignetes textiles Gewebe oder Gestricke verwendet werden, wobei die definierten Wirkflächen oder Wirkbereiche durch verstärkte Areale gebildet sein können, entweder mit engerer Maschenweite oder durch eingearbeitete Mehrfachlagen des textilen Materials oder durch andere geeignete Verstärkungsmittel, so dass diese verstärkten Wirkbereiche als Ankerflächen dienen können, um einerseits die von den flexiblen und/oder elastischen Abschnitten oder Bändern eingeleiteten Kräfte in den Körper des Patienten weiterleiten zu können und um andererseits den flexiblen und/oder elastischen Abschnitten oder Bändern jeweils einen ausreichend stabilen Verankerungspunkt zu bieten.

Ebenso denkbar ist jedoch eine Ausführungsvariante, bei denen die als Verankerungsflächen für die Zugelemente dienenden Abschnitte im Wesentlichen unverstärkt sind, sondern lediglich mit geeigneten Haftmitteln zur insbesondere lösbaren Verankerung der Zugelemente ausgestattet sind.

Die flexiblen und/oder elastischen Abschnitte oder Zugelemente können zumindest einseitig auf dem textilen Material des Oberbekleidungsstückes oder Rumpfkorsetts aufgebracht sein und/oder auf dessen Oberfläche verlaufen. Mit Oberfläche des Oberbekleidungsstückes oder textilen Rumpfkorsetts kann hierbei die vom Patienten abgewandte Außenseite gemeint sein, die beim Tragen des Oberbekleidungsstückes oder Rumpfkorsetts sichtbar ist. Mit dem Begriff der Oberfläche kann jedoch auch die dem Patienten zugewandte Innenseite des Oberbekleidungsstückes oder Rumpfkorsetts gemeint sein, die beim Tragen des Oberbekleidungsstückes oder textilen Rumpfkorsetts sind sichtbar ist.

Eine optionale, aber vorteilhafte Variante des Oberbekleidungsstückes oder textilen Rumpfkorsetts kann vorsehen, dass zumindest einer der flexiblen und/oder elastischen Abschnitte oder Zugelemente vom textilen Trägermaterial gelöst und/oder in seiner Position verändert werden kann oder dass die Abschnitte - im Falle von mehreren flexiblen und/oder elastischen Abschnitten - in ihren Positionen verändert werden können.

In diesem Zusammenhang kann es außerdem von Vorteil sein, wenn der wenigstens eine vom textilen Trägermaterial lösbare flexible und/oder elastische Abschnitt mittels Klettverbindungen oder anderer geeigneter lösbarer Verbindungstechnik am textilen Trägermaterial fixiert oder bedarfsweise wieder von diesem gelöst werden kann. Diese Lösbarkeit kann insbesondere dazu genutzt werden, den wenigstens einen flexiblen und/oder elastischen Abschnitt oder das wenigstens eine Zugelement entweder in seiner Position verändern oder auch gegen einen Abschnitt oder gegen ein Zugelement anderer Flexibilität austauschen zu können.

Allerdings kann es ebenso sinnvoll sein, wenn bei einer alternativen Variante des erfindungsgemäßen Oberbekleidungsstückes oder textilen Rumpfkorsetts die flexiblen und/oder elastischen Abschnitte oder Zugelemente in das textile Material des Oberbekleidungsstückes oder Rumpfkorsetts eingearbeitet sind und/oder zumindest teilweise mit diesem in stoffschlüssiger Verbindung stehen. Damit kann etwa gemeint sein, dass die flexiblen und/oder elastischen Abschnitte oder Zugelemente auf das textile Material aufgeklebt und/oder bereichsweise mit diesem verschweißt oder auf andere Weise verbunden sind.

Auch wenn die Mindestanzahl an Wirkflächen und/oder Wirkbereichen zwei solche voneinander beabstandete Flächen oder Bereiche sind, die durch mindestens ein flexibles oder elastisches Band miteinander verbunden und gekoppelt sind, so können bei dem Oberbekleidungsstück oder textilen Rumpfkorsett durchaus aus drei oder auch deutlich mehr Wirkflächen und/oder Wirkbereiche vorgesehen sein, die untereinander durch gleich dimensionierte oder durch unterschiedliche flexible und/oder elastische Abschnitte oder Zugelemente miteinander gekoppelt sind, wobei diese Bänder oder Abschnitte jeweils gleiche oder unterschiedliche Rückstell- und/oder Spannkräfte ausbilden können, so dass eine für den jeweiligen Patienten einstellbare individuelle Wirkung erzielt werden kann. Die unterschiedlichen Flexibilitäten der flexiblen und/oder elastischen Abschnitte, Zugelemente oder Bänder können bspw. farbcodiert sein, so dass etwa durch unterschiedliche Bandfarben eine Reihe von definierten Bandstärken oder Flexibilitäten zur Verfügung gestellt werden kann.

Es sollte an dieser Stelle betont werden, dass das erfindungsgemäße Oberbekleidungsstück oder textile Korsett seine therapeutischen Wirkungen auf den betroffenen Patienten nicht nur durch die Gestaltung, Dimensionierung und Positionierung der zwei oder mehr Wirkflächen entfaltet, sondern insbesondere durch die Auswahl und Dimensionierung der die Wirkflächen verbindenden und Zugkräfte zwischen ihnen erzeugenden Band- und/oder Streifenabschnitte, da nur durch das Zusammenwirken aller Komponenten die gewünschten Kräfte und rückstellenden Momente auf die verkrümmte und/oder verdrehte Wirbelsäule des Patienten erzeugt werden können.

Eine weitere Variante des erfindungsgemäßen Oberbekleidungsstückes oder textilen Rumpfkorsetts kann dadurch charakterisiert sein, dass die elastischen und/oder flexiblen Abschnitte durch unverzweigte oder verzweigte elastische Zugbänder gebildet sind, bei denen sich mindestens ein Abschnitt, der zwischen zwei Wirkflächen verläuft und diese zugkraftübertragend verbindet, in einen weiteren Abschnitt verzweigt, dessen Ende entweder an anderer Stelle mit einer der beiden zugkraftübertragend verbundenen Wirkflächen oder mit einer weiteren Wirkfläche verbunden und dort verankert ist.

Wahlweise können auch derartige verzweigte Zugbänder vielfältige Gestaltungen aufweisen und durch die Stärke, die Länge, die Rückstellkräfte und die Konturen der Zugbänder sowie deren Zweigabschnitte individuell an das Krankheitsbild und die besondere Situation des jeweiligen Patienten angepasst werden.

Außerdem können auch die Zweigabschnitte eines solchen verzweigten Zugbandes unterschiedlich breit ausgeführt sein, wobei die Zweigabschnitte nahezu beliebig schräg oder diagonal am Körper verlaufen können. Auf diese Weise kann auch ein solches einfach oder wahlweise auch mehrfach verzweigtes Zugband die hier angestrebte Aufgabe erfüllen, mittels der Auswahl der passend dimensionierten einfachen oder verzweigten Zugbänder, die jeweils unterschiedliche Elastizitäten aufweisen und damit veränderliche Zug- und Rückstellkräfte zwischen den miteinander verbundenen Wirkflächen erzeugen, dem textilen Rumpfkorsett die für den jeweiligen Patienten passenden Stützkräfte aufzuprägen, die es dem Patienten erleichtern, eine verbesserte aufrechte Körperhaltung einzunehmen, ohne dass er hierfür zwingend ein steifes Halbschalenkorsett tragen müsste.

Unterschiedliche verzweigte Zugbänder mit jeweils unterschiedlichen Stärken und von ihnen erzeugten Zugkräften können bspw. unterschiedliche Farben aufweisen und solchermaßen kodiert sein, um bei der Anpassung des textilen Rumpfkorsetts an den Träger eine Auswahl aus einer begrenzten Palette von verzweigten oder unverzweigten Zugbändern treffen zu können.

Auch bei der Variante mit verzweigten Zugbändern kann ggf. auf verstärkte Wirkflächen verzichtet werden, wobei diese verzweigten Zugbänder insbesondere mit dem textilen Material des Shirts verklebt, verschweißt oder vernäht sein können. Die von den Zugbändern erzeugten Zugkräfte können ggf. auch ohne Unterstützung der beschriebenen Wirkflächen in das Oberbekleidungsstück oder textile Rumpfkorsett eingeleitet werden.

Eine weitere Variante eines erfindungsgemäßen Oberbekleidungsstückes oder textilen Rumpfkorsetts steht zumindest in einigen Aspekten im Widerspruch zu einigen anderen der oben genannten Varianten, soll aber gleichwohl Teil der Erfindung sein und eine Priorität begründen, da diese Variante vom wesentlichen Erfindungsgedanken gleichermaßen Gebrauch macht und sich nur in einigen Details vom Gegenstand einiger der abhängigen Ansprüche unterscheidet.

So kann auch bei dieser Variante das erfindungsgemäße Oberbekleidungsstück oder textile Rumpfkorsett vorzugsweise einem Oberbekleidungsstück ähneln und bspw. durch ein Bekleidungsstück ähnlich einem Pullover oder einem kurzärmeligen Shirt o. dgl. gebildet sein, wie dies bereits oben erläutert wurde. Wirkflächen, typischerweise gebildet jeweils durch verstärkte textile Bereiche, können wahlweise vorhanden sein, müssen aber nicht vorhanden sein.

Sofern dort solche durch verstärkte textile Bereiche gebildeten Wirkflächen vorhanden sind, können diese insbesondere durch ihre Integration in das textile Material des Shirts jeweils flächig an den jeweiligen Rumpfbereichen des Patienten auf- oder anliegen. Wahlweise kann auch die hier erläuterte Ausführungsvariante des textilen Rumpfkorsetts Haftflächen, bspw. gebildet durch Klettflächen o. dgl., aufweisen, um mindestens einen flexiblen Abschnitt dort zu verankern.

Das hier ergänzend erläuterte und sich nicht exakt im Wortlaut aller abhängigen Ansprüche wiederfindende Oberbekleidungsstück oder textile Rumpfkorsett ist im Wesentlichen durch ein breiteres Zugband charakterisiert, das den flexiblen Abschnitt bildet. Das breitere Zugband verläuft quer über den Rücken und/oder die vordere Rumpfseite des Patienten und ist mit seinen beiden Enden an der Frontseite bzw. an der Rückseite des Shirts oder der textilen Oberbekleidung befestigt, wahlweise über Haftflächen, durch eine komplette oder abschnittsweise verlaufende Verankerung und/oder Integration in das textile Material des Shirts. Denkbar ist auch eine Variante, bei der die normalerweise voneinander beabstandeten Enden des breiteren Zugbandes aneinander stoßen und/oder überlappen oder miteinander verbunden sind, so dass eine geschlossene Schlaufe gebildet ist, die horizontal oder abschnittsweise diagonal um den Körper des Patienten herum verläuft.

Wie dies schon zuvor anhand der oben erläuterten Ausführungsvarianten betont wurde, kann auch ein solches breiteres Zugband vielfältige Gestaltungen aufweisen und durch die Stärke, die Länge, die Rückstellkräfte und die Konturen des Zugbandes sowie dessen Verlauf über der Oberfläche des Shirts individuell an das Krankheitsbild und die besondere Situation des jeweiligen Patienten angepasst werden. Es muss deshalb an dieser Stelle nicht betont werden, dass das hier erläuterte Ausführungsbeispiel nicht einschränkend zu verstehen ist, sondern lediglich die der Erfindung zugrunde liegenden Prinzipien der Korrektor von Fehlhaltungen des Körperrumpfes des Patienten verdeutlichen soll.

Das breitere Zugband kann unterschiedlich breit ausgeführt sein, wobei der Verlauf nahezu beliebig schräg oder diagonal am Körper entlang führen kann. Auf diese Weise kann auch ein solches breiteres Zugband, das ggf. auch einfach oder wahlweise auch mehrfach verzweigt sein kann, die hier angestrebte Aufgabe erfüllen, mittels der Auswahl des passend dimensionierten einfachen oder verzweigten breiteren Zugbandes, das angepasste und/oder unterschiedliche Elastizitäten aufweisen und damit veränderliche Zug- und Rückstellkräfte zwischen den miteinander verbundenen Wirkflächen erzeugen kann, dem textilen Rumpfkorsett die für den jeweiligen Patienten passenden Stützkräfte aufzuprägen, die es dem Patienten erleichtern, eine verbesserte aufrechte Körperhaltung einzunehmen, ohne dass er hierfür zwingend ein steifes Halbschalenkorsett tragen müsste.

Zur Erreichung zumindest eines Teils der oben genannten Ziele schlägt die vorliegende Erfindung neben dem in zahlreichen Ausführungsvarianten beschriebenen Oberbekleidungsstück oder textilen Rumpfkorsett zudem ein Verfahren zur Anpassung und/oder Variation von auf einen Oberkörper eines Trägers einwirkenden Stütz- und/oder Zugkräften mittels eines Oberbekleidungsstückes vor, welches insbesondere der Abstützung und/oder zur therapeutischen Behandlung von Wirbelsäulenstellungen, die von einer aufrechten Normalstellung abweichen, dienen kann. Diese Abweichungen oder Fehlstellungen stehen insbesondere im Zusammenhang mit dem Krankheitsbild einer Skoliose, wie dies bereits oben ausführlich erläutert wurde.

Zwischen mindestens zwei voneinander entfernten Abschnitten des Oberbekleidungsstückes befindet sich jeweils mindestens ein zumindest einseitig von einem der mindestens zwei Abschnitte lösbares Zugelement. Dieses mindestens eine Zugelement verbindet in einem mit den beiden entfernten Abschnitten jeweils verbundenen Zustand die mindestens zwei voneinander beabstandeten Abschnitte und erzeugt dabei gleichzeitig definierte oder definierbare Zugkräfte zwischen den mindestens zwei Abschnitten.

Das mindestens eine Zugelement verfügt über elastische Eigenschaften, die sich von den elastischen Eigenschaften des Oberbekleidungsstückes dahingehend unterscheiden, dass die elastischen Rückstellkräfte des mindestens einen Zugelementes höher sind als diejenigen Bereiche des Oberbekleidungsstückes, die sich zwischen den mindestens zwei Abschnitten befinden.

Außerdem sieht das Verfahren vor, dass die auf das Oberbekleidungsstück und damit auf den Oberkörper des Trägers einwirkenden Stütz- und/oder Zugkräfte durch Variation einer Positionierung des mindestens einen Zugelementes zwischen den mindestens zwei Abschnitten und/oder durch dessen zumindest einseitiges Lösen von einem der Abschnitte verändert werden können.

Wahlweise können die Zugelemente umgesetzt und so zwischen jeweils voneinander beabstandeten Abschnitten aufgesetzt werden, dass ihre jeweilige wirksame Länge variiert. Auf diese Weise können die Zugelemente stärker vorgespannt und solchermaßen höhere Zugkräfte ausgeübt werden, die auf den Träger zur Beeinflussung seiner Körperhaltung wirken.

Ebenso können bedarfsweise die Zugelemente gegen solche anderer Länge und/oder anderer elastischer Eigenschaften ausgetauscht werden, wodurch ebenfalls die wirksamen Zugkräfte verändert und bedarfsweise angepasst werden können.

Eine vorteilhafte Variante des erfindungsgemäßen Verfahrens kann eine optische Vermessung des mit dem Oberbekleidungsstück bekleideten Trägers und seiner Oberkörperposition sowie dessen Stellung und/oder Position vorsehen. Bei der Vermessung des mit dem Oberbekleidungsstück bekleideten Trägers variiert seine Oberkörperposition sowie seine Stellung und/oder Position unter der Wirkung mindestens eines zwischen zwei voneinander entfernten Abschnitten platzierten Zugelementes und damit unter der Wirkung der von dem mindestens einen Zugelement zwischen den voneinander entfernten Verankerungsflächen ausgeübten Zugkräften.

Mittels der optischen Vermessung kann die Wirksamkeit jeder Maßnahme, d.h. die Wirksamkeit jeder Applikation einzelner Zugelemente überprüft und verifiziert werden.

Die optische Vermessung kann insbesondere unter Erfassung von optisch erkennbaren Referenzpunkten am Oberbekleidungsstück erfolgen, was ein hohes Maß an Reproduzierbarkeit jedes Messvorganges gewährleisten kann.

Sinnvollerweise sind die Referenzpunkte jeweils den Abschnitten am Oberbekleidungsstück zugeordnet.

Außerdem können bei dem Verfahren mehrere optische Vermessungen nach Anpassung und/oder Austausch der Zugelemente und/oder unter Variation der Positionen mindestens eines Zugelementes oder mehrerer Zugelemente durchgeführt werden, so dass eine optimierte Wirkung erzielt und gleichzeitig überprüft werden kann.

Der flexible und/oder elastische Abschnitt, der durch das breitere Zugband gebildet ist, kann durch ein fest verankertes, außen auf dem Shirt aufgebrachtes und dessen Wirkflächen unter Herstellung von definierten Zugkräften zwischen diesen verbindendes breiteres Zugband oder durch einen in das textile Material des Shirts eingearbeiteten flexiblen und/oder elastischen Abschnitt gebildet sein. Auch für diese Variante gilt das zuvor gesagte, nach dem das breitere Zugband bspw. mit dem textilen Material des Shirts verklebt, verschweißt oder vernäht sein kann. Die vom Zugband erzeugten Zugkräfte können ggf. auch ohne Unterstützung der beschriebenen Wirkflächen in das textile Rumpfkorsett eingeleitet werden.

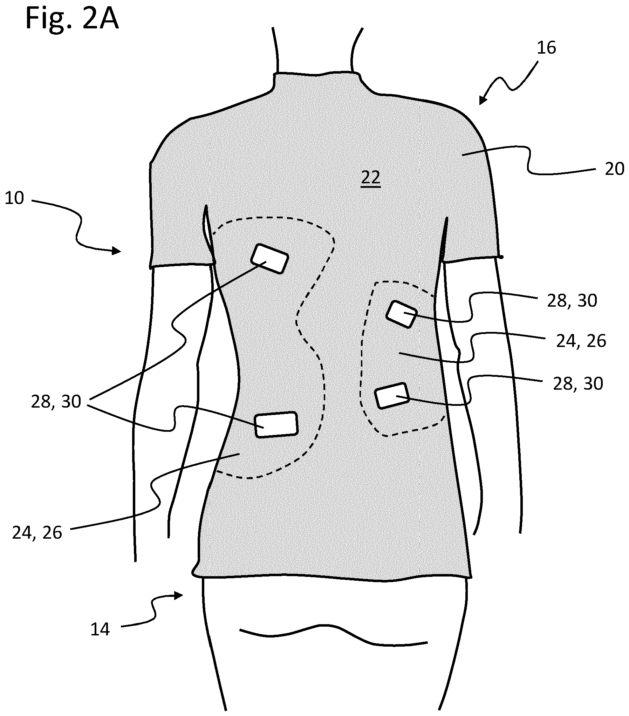

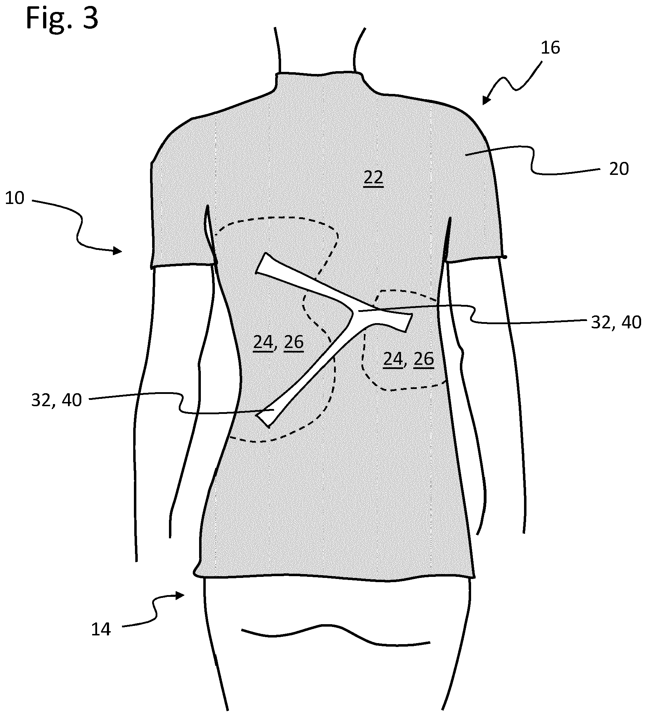

Ein Verfahren zur Anpassung und Herstellung eines solchen textilen Rumpfkorsetts findet sich in der Beschreibung eines Ausführungsbeispiels weiter unten, wobei auf die Figuren 2A bis 2F sowie ergänzend auf die Figuren 3, 4 und 5 Bezug genommen wird. Es sei an dieser Stelle betont, dass das anhand der Figuren 2A bis 2F (vgl. auch ergänzend Fig. 3, Fig. 4 und Fig. 5) im Detail und in unterschiedlichen Varianten erläuterte Verfahren zur Anpassung des textilen Rumpfkorsetts an einen Patienten und zur Herstellung solcher Rumpfkorsetts Bestandteil der hier ausgebreiteten Erfindung ist und dass die dort für den Fachmann erkennbaren Erfindungsaspekte jeweils prioritätsbegründend sein sollen.

Es sei an dieser Stelle ausdrücklich erwähnt, dass alle Aspekte und Ausführungsvarianten, die im Zusammenhang mit dem erfindungsgemäßen Oberbekleidungsstück erläutert wurden, gleichermaßen Teilaspekte des erfindungsgemäßen Verfahrens betreffen oder bilden können. Wenn daher an einer Stelle bei der Beschreibung oder auch bei den Anspruchsdefinitionen zum erfindungsgemäßen Oberbekleidungsstück von bestimmten Aspekten und/oder Zusammenhängen und/oder Wirkungen die Rede ist, so gilt dies gleichermaßen für das erfindungsgemäße Verfahren. In umgekehrter Weise gilt dasselbe, so dass auch alle Aspekte und Ausführungsvarianten, die im Zusammenhang mit dem erfindungsgemäßen Verfahren erläutert wurden, gleichermaßen Teilaspekte des erfindungsgemäßen Oberbekleidungsstückes betreffen oder sein können. Wenn daher an einer Stelle bei der Beschreibung oder auch bei den Anspruchsdefinitionen zum erfindungsgemäßen Verfahren von bestimmten Aspekten und/oder Zusammenhängen und/oder Wirkungen die Rede ist, so gilt dies gleichermaßen für das erfindungsgemäße Oberbekleidungsstück.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Fig. 1 zeigt ein herkömmliches Rumpf-Korsett gemäß bekanntem Stand der Technik.
Fig. 2 zeigt in insgesamt sechs schematischen Ansichten (Fig. 2A, Fig. 2B, Fig. 2C, Fig. 2D, Fig. 2E und Fig. 2F) eine Ausführungsvariante eines Oberbekleidungsstückes oder textilen Rumpfkorsetts gemäß vorliegender Erfindung sowie aufeinanderfolgende Verfahrensschritte zur Herstellung eines solchen textilen Rumpfkorsetts.
Fig. 3 zeigt eine weitere Ausführungsvariante des Oberbekleidungsstückes oder textilen Rumpfkorsetts gemäß vorliegender Erfindung.
Fig. 4 zeigt eine weitere alternative Ausführungsvariante des Oberbekleidungsstückes oder textilen Rumpfkorsetts gemäß vorliegender Erfindung.
Fig. 5 zeigt eine Rückseite eines Oberbekleidungsstückes, das mit zahlreichen Verankerungsflächen zum lösbaren Applizieren von elastischen Zugelementen ausgestattet ist.

Für gleiche oder gleich wirkende Elemente der Erfindung werden in den nachfolgend erläuterten Zeichnungen jeweils gleiche Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie die Erfindung ausgestaltet sein kann und stellen keine abschließende Begrenzung dar. Auch sind die nachfolgend beschriebenen Merkmale jeweils nicht in engem Zusammenhang mit weiteren Merkmalen des jeweiligen Ausführungsbeispiels zu verstehen, sondern können jeweils im allgemeinen Zusammenhang vorgesehen sein bzw. hierfür Verwendung finden.

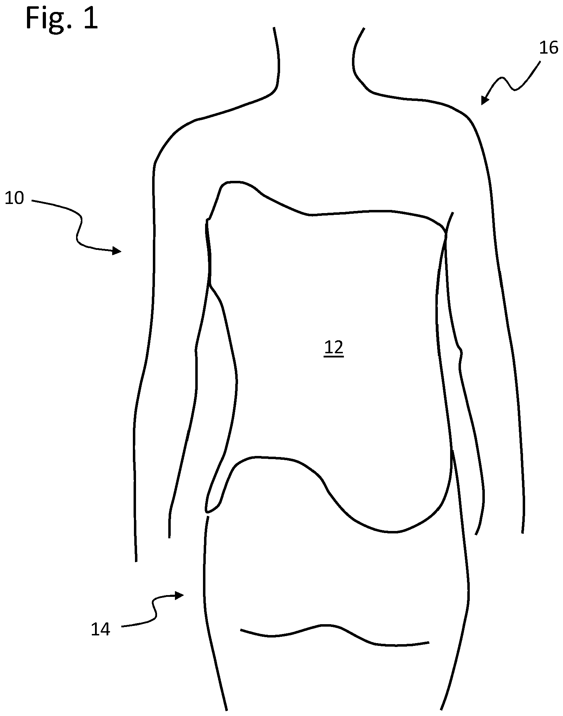

Die Fig. 1 zeigt anhand einer stark schematisierten Rückansicht eines aufrechtstehenden Patienten ein herkömmliches Rumpf-Korsett, wie es dem bekannten Stand der Technik entspricht. Der Patient ist hier allgemein mit der Bezugsziffer 10 gekennzeichnet, während das Korsett, bei dem es sich insbesondere um ein sog Chêneau-Korsett handeln kann, allgemein mit der Bezugsziffer 12 gekennzeichnet ist. In Fig. 1 ist allerdings aufgrund der rückseitigen Darstellung des Patienten 10 auch nur die Rückseite des normalerweise aus zwei Halbschalen gebildeten Korsetts 12 erkennbar. Ein solches Korsett 12 kann normalerweise an einer vertikalen Längsseite geöffnet und an der gegenüberliegenden vertikalen Längsseite aufgeklappt werden, um es anzulegen.

Ein solches Chêneau-Korsett oder allgemein Rumpf-Korsett 12 weist relativ große Halbschalen auf, die von der Hüfte 14 bis zumindest unterhalb der Schultern 16 reichen. Die Größe der Halbschalen verursacht einen relativ großen Fertigungs- und Anpassungsaufwand für ein solches Korsett, zumal an den innenliegenden Oberflächen der Halbschalen normalerweise noch mehrere Druckpolster anzubringen sind, die in Fig. 1 jedoch nicht erkennbar sind.

Die insgesamt sechs schematischen Darstellungen der Figuren 2A bis 2F verdeutlichen aufeinander folgende Prozessphasen mehrerer möglicher Varianten eines Verfahrens zur Herstellung erfindungsgemäßer textiler Rumpfkorsetts 20 anhand von schematisierten Rückansichten eines jeweils aufrechtstehenden Patienten 10.

Das erfindungsgemäße textile Rumpfkorsett 20 kann vorzugsweise einem Oberbekleidungsstück ähneln und bspw. durch ein Bekleidungsstück ähnlich einem Pullover oder einem kurzärmeligen Shirt gebildet sein. Das erfindungsgemäße textile Rumpfkorsett 20 kann jedoch auch durch ein solches Oberbekleidungsstück 22 gebildet sein, welches bspw. durch einen Pullover oder ein kurzärmeliges Shirt gebildet sein kann. Im hier vorgestellten Ausführungsbeispiel ist das textile Rumpfkorsett 20 durch ein solches Oberbekleidungsstück oder Shirt 22 gebildet, das mindestens zwei Abschnitte oder Wirkflächen 24 aufweist, die vorzugsweise durch verstärkte textile Bereiche 26 gebildet sein können.

Diese verstärkten textilen Bereiche 26, Abschnitte oder Wirkflächen 24 sind in den Figuren 2A bis 2F jeweils durch unterbrochene Linien angedeutet. Eine linke Wirkfläche 24 ist hierbei etwas größer und befindet sich oberhalb der Hüfte 14 auf der linken Körperhälfte des Patienten 10. Eine etwas kleinere Wirkfläche 24 befindet sich ebenfalls auf der Hüfte, jedoch auf der rechten Körperhälfte des Patienten 10. Die linken und rechten Wirkflächen sind jeweils am Rückenbereich als auch am hier nicht sichtbaren vorderen Körperbereich voneinander beabstandet.

Die Figuren 2A bis 2F zeigen ein Ausführungsbeispiel mit zwei solchen Abschnitten oder Wirkflächen 24, die jeweils durch verstärkte textile Bereiche 26 gebildet sind, die durch ihre Integration in das textile Material des Oberbekleidungsstückes oder Shirts 22 jeweils flächig an den gezeigten linken und rechten Rumpfbereichen des Patienten 10 auf- oder anliegen. Beim gezeigten Ausführungsbeispiel bedecken die zwei Wirkflächen 24 weniger als die Hälfte einer Gesamtoberfläche des Rumpfbereichs des Patienten 10 zwischen dessen Beckenbereich und dessen Halsbereich.

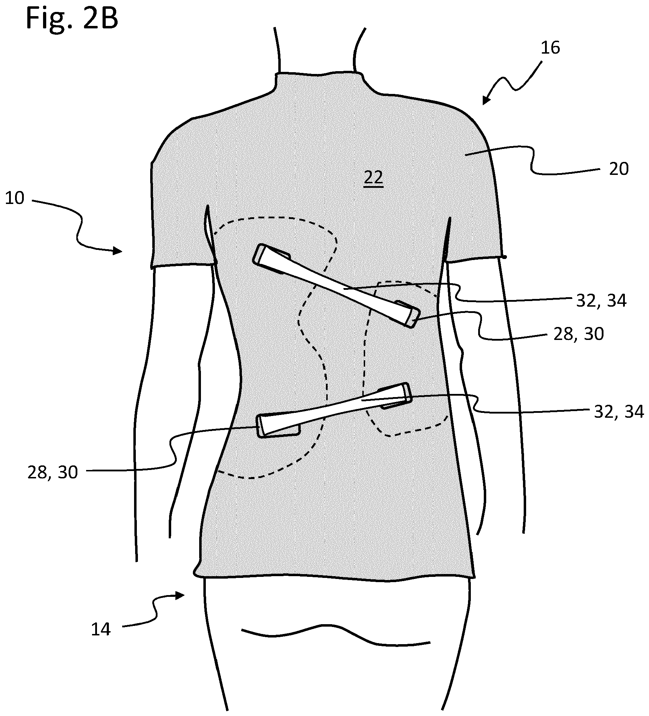

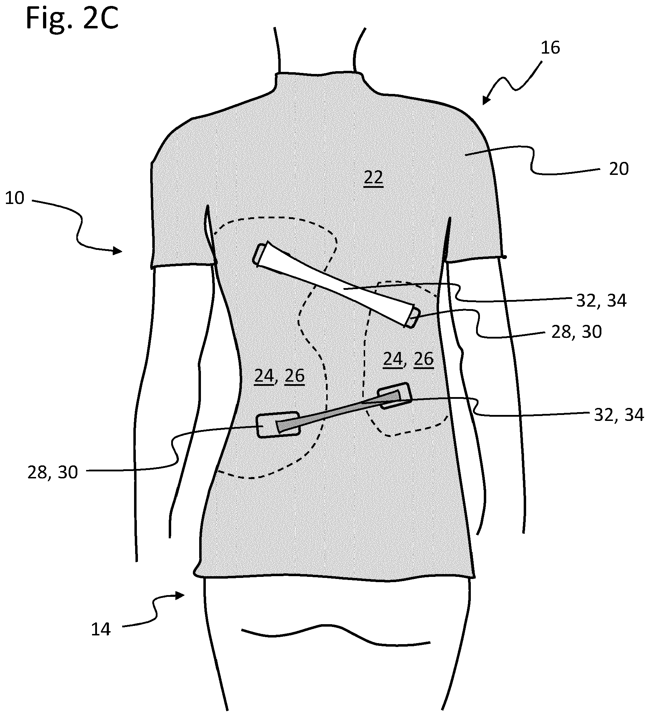

Die schematische Rückansicht der Fig. 2A lässt weiterhin mehrere Haftflächen 28 erkennen, die bspw. durch Klettflächen 30, durch Schnallen, durch Druckknöpfe, Schlaufen o. dgl. mehr gebildet sein können. Die Haftflächen 28 dienen der temporären oder dauerhaften Verankerung von flexiblen und/oder elastischen Abschnitten 32 (Fig. 2B, Fig. 2C), die definierte Zugkräfte zwischen den voneinander beabstandeten Wirkflächen 24 erzeugen sollen, was der angestrebten Korrektur der Körperhaltung des Patienten dienen soll.

Während die durch die verstärkten textilen Bereiche 26 gebildeten Wirkflächen 24 jeweils eine sinnvolle Ausdehnung zwischen etwa 400 cm³ und etwa 0,7 ... 1,0 m³ aufweisen können, sind demgegenüber die Haftflächen 28 relativ klein. Im Falle von Klettflächen 30 können die Haftflächen 28 bspw. jeweils eine sinnvolle Größe von etwa 3 cm³ bis über 20 cm³ aufweisen.

Sofern es sich bei dem Shirt 22 um ein reines Testshirt zur Ermittlung der sinnvollen Positionen der flexiblen und/oder elastischen Abschnitte 32 und der Auswahl und Dimensionierung geeigneter flexibler und/oder elastischer Abschnitte 32 handelt, das nicht zur späteren Dauerverwendung durch den Patienten 10 vorgesehen ist, können die Haftflächen 28 oder Klettflächen 30 jedoch auch deutlich größer gewählt werden, als dies in Fig. 2A und den Figuren 2B ff. beispielhaft gezeigt ist.

Aus dem Gesagten wird deutlich, dass der in Fig. 2A gezeigte Fertigungs- oder Vorbereitungszustand des textilen Rumpfkorsetts 20 oder Oberbekleidungsstückes 22 lediglich den Ausgangszustand bildet, der es gemäß Fig. 2B sowie gemäß Fig. 2C ermöglicht, mittels der an den Klettflächen 30 oder Haftflächen 28 verankerten flexiblen und/oder elastischen Abschnitte 32 definierte und veränderliche Zugkräfte in die durch die verstärkten textilen Bereiche 26 gebildeten Wirkflächen 24 einzuleiten, was wiederum Stütz- und Haltekräfte erzeugte, die es dem Patienten 10 als Träger des solchermaßen ausgestatteten Shirts 22 erleichtern können, eine der zu behandelnden Wirbelsäulenverkrümmung (Skoliose) entgegenwirkende verbesserte Körperhaltung einzunehmen.

Die hier ganz allgemein als flexible elastische Abschnitte 32 bezeichneten elastischen Zugbänder 34 - im vorliegenden Zusammenhang auch als Zugelemente 34 bezeichnet - weisen an ihren flachen Enden jeweils Kontaktflächen auf, die mit den Haftflächen 28 oder Klettflächen 30 korrespondieren und eine feste, aber bedarfsweise lösbare Verankerung der Zugelemente oder Zugbänder 34 auf den jeweiligen Haftflächen 28 oder Klettflächen 30 benachbarter, aber voneinander beabstandeter Wirkflächen 24 erlauben. Im Falle von Klettflächen 30 auf den verstärkten textilen Bereichen 26 weisen die Zugelemente oder Zugbänder 34 entsprechende Gegenflächen auf, die mit den Häkchen oder Schlaufen der als Klettflächen 30 ausgebildeten Haftflächen 28 korrespondieren und mit diesen eine belastbare, jedoch bedarfsweise lösbare Verbindung bilden.

Die Fig. 2B lässt eine Variante mit zwei in etwa gleich breiten Zugelementen oder Zugbändern 34 erkennen, wobei das obere Zugelement oder Zugband 34 aufgrund der weiter voneinander entfernten oberen Haftflächen 28 der linken und rechten Wirkfläche 24, die mittels des oberen Zugbandes 34 miteinander verbunden werden, etwas länger ist als das untere Zugelement oder Zugband 34. Die Fig. 2B lässt zudem erkennen, dass die jeweils mittels einzelner Zugelemente oder Zugbänder 34 verbundenen Haftflächen 28 auf unterschiedlichen Höhenniveaus angeordnet sein können, so dass sich schräge oder diagonale Verläufe der Zugbänder 34 ergeben können.

Die Fig. 2C lässt eine leicht modifizierte Variante mit zwei unterschiedlich breiten Zugelementen oder Zugbändern 34 erkennen, wobei das breitere obere Zugelement oder Zugband 34 aufgrund der weiter voneinander entfernten oberen Haftflächen 28 der linken und rechten Wirkfläche 24, die mittels des oberen Zugbandes 34 miteinander verbunden werden, etwas länger ist als das schmäler ausgebildete untere Zugelement oder Zugband 34. Auch bei der Variante gemäß Fig. 2C sind die jeweils mittels einzelner Zugelemente oder Zugbänder 34 verbundenen Haftflächen 28 auf unterschiedlichen Höhenniveaus angeordnet, so dass sich auch hier schräge oder diagonale Verläufe der Zugbänder 34 ergeben.

Der Vergleich der Figuren 2B und 2C macht deutlich, dass für die exakte Anpassung des erfindungsgemäßen textilen Rumpfkorsetts 20 oder Oberbekleidungsstückes 22 nicht nur die Gestaltung, die Größe und die Materialstärke der Wirkflächen 24 oder verstärkten textilen Bereiche 26 wichtig ist. Vielmehr ist es von besonderer Bedeutung, mittels der Auswahl der passenden Zugelemente oder Zugbänder 34, die jeweils unterschiedliche Elastizitäten aufweisen und damit veränderliche Zug- und Rückstellkräfte zwischen den miteinander verbundenen Wirkflächen 24 erzeugen, dem textilen Rumpfkorsett 20 die für den jeweiligen Patienten 10 passenden Stützkräfte aufzuprägen, die es dem Patienten erleichtern, eine verbesserte aufrechte Körperhaltung einzunehmen, ohne dass er hierfür zwingend ein steifes Halbschalenkorsett tragen müsste.

Unterschiedliche Zugelemente oder Zugbänder 34 mit jeweils unterschiedlichen Stärken und von ihnen erzeugten Zugkräften können bspw. unterschiedliche Farben aufweisen und solchermaßen kodiert sein, um bei der Anpassung des textilen Rumpfkorsetts 20 an den Träger eine Auswahl aus einer begrenzten Palette von Zugbändern 34 treffen zu können.

Auch wenn die Figuren 2A bis 2F nur jeweils die Rückseite des durch das Shirt 22 gebildeten textilen Rumpfkorsetts 20 zeigen, ist es wichtig zu betonen, dass sich an der Vorderseite des Rumpfkorsetts 20 entweder eine ähnliche Konfiguration mit mindestens einem Zugband 34 oder einem anderen geeigneten flexiblen und/oder elastischen Abschnitt zur Verbindung der auch an der Vorderseite voneinander beabstandeten Wirkflächen 24 befindet, oder dass die rückseitig voneinander beabstandeten Wirkflächen 24 an der Vorderseite des Shirts 22 ineinander übergehen oder miteinander verbunden sind. Je nach individueller Ausprägung des zu behandelnden Krankheitsbildes des Patienten 10 kann die eine oder andere Variante sinnvoll eingesetzt werden. Während die erstgenannte Variante mit den vorderseitig angeordneten lösbaren und austauschbaren Zugbändern 34 umfangreichere Anpassungs- und Einstellungsmöglichkeiten bieten, kann die alternative Variante ohne Zugbänder und mit stattdessen vorderseitig verbundenen Wirkflächen für manche schwächere Ausprägungen von Wirbelsäulenfehlstellungen eine vereinfachte Anpassung und günstigere Fertigung des textilen Rumpfkorsetts ermöglichen.

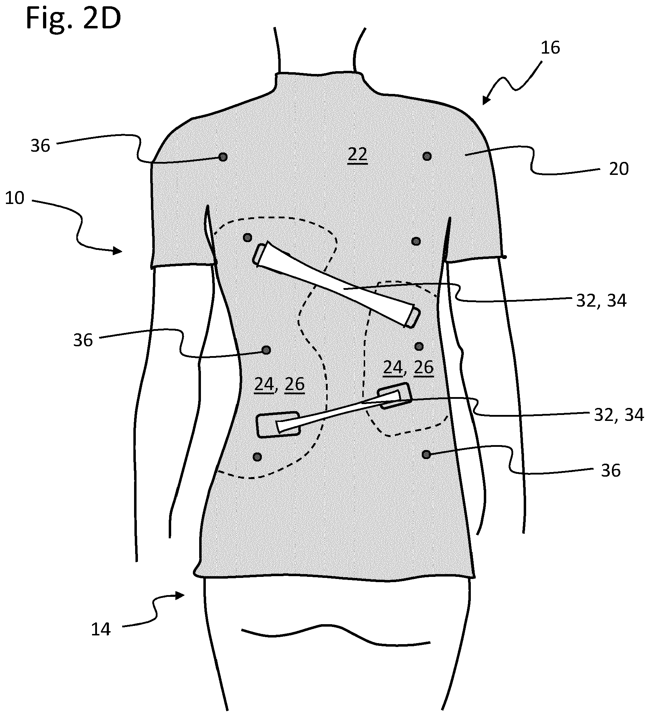

Die Fig. 2D verdeutlicht einen weiteren optionalen Prozessschritt bei der Herstellung eines individuell angepassten textilen Rumpfkorsetts 20. Auf der Rückseite des Shirts 22 befinden sich mehrere optisch erfassbare Referenzpunkte 36, die bspw. mittels einer hier nicht gezeigten Kamera mit nachgeschalteter Bildauswertung die Auswirkungen unterschiedlicher Zugbänder 34 auf die Körperhaltung des Patienten erkennen und verarbeiten können. Durch Austausch der Zugbänder 34 und Auswahl der jeweils passenden kann die Körperhaltung und die Stützwirkung des Rumpfkorsetts 20 solange verändert und korrigiert werden, bis eine zufriedenstellende Lösung gefunden wurde, wobei durch die optische Erfassung der Referenzpunkte 36 und die Verarbeitung der gewonnenen Bilddaten eine Erfassung und Speicherung der für den jeweiligen Patienten sinnvollen Korrekturdaten ermöglicht ist.

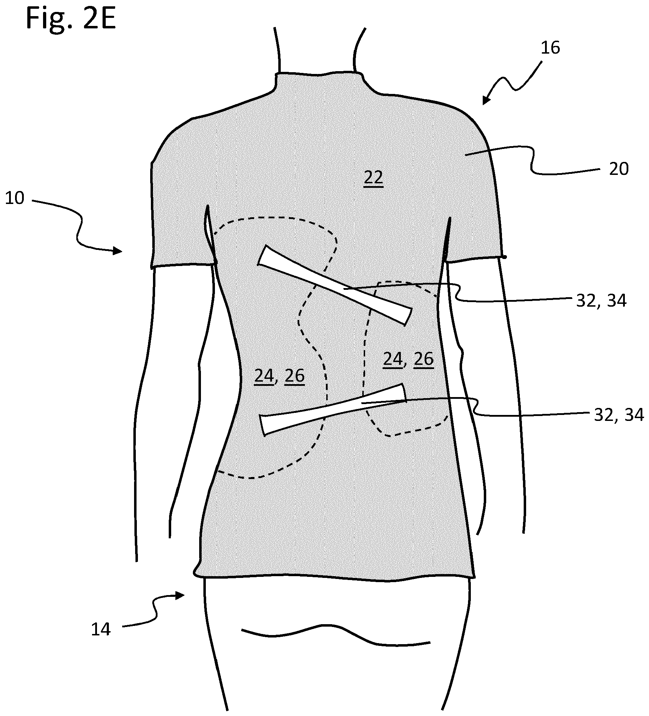

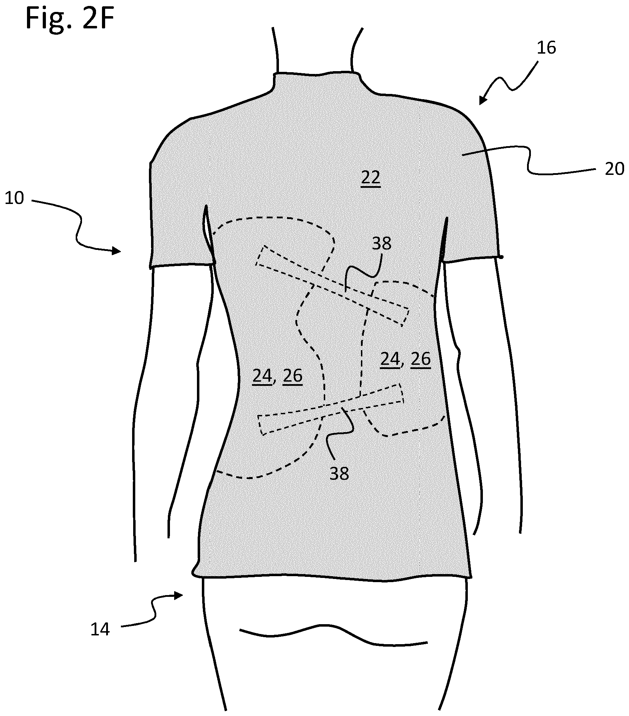

Da es im Interesse einer zuverlässigen Korrektur und einer Beibehaltung der zuvor ermittelten Zugkräfte, die durch die Zugbänder 34 aufgebracht werden, wünschenswert ist, die zuvor durch korrespondierende Haftflächen lösbaren und austauschbaren Zugbänder 34 fest und unlösbar am Shirt 22 zu verankern, kann bspw. auf Grundlage der mittels der Referenzpunkte 36 (vgl. Fig. 2D) überprüften Einstell- und Korrekturdaten eine maschinelle oder manuell unterstützte teilmaschinelle Fertigung eines textilen Rumpfkorsetts 20 gemäß Fig. 2E oder gemäß Fig. 2F erfolgen.

Bei diesen beiden Varianten sind die flexiblen und/oder elastischen Abschnitte 32 entweder gemäß Fig. 2E durch fest verankerte, außen auf dem Shirt 22 aufgebrachte und dessen Wirkflächen 24 unter Herstellung von definierten Zugkräften zwischen diesen verbindende Zugbänder 34 oder durch in das textile Material des Shirts 22 eingearbeitete flexible und/oder elastische Abschnitte 32 gemäß Fig. 2F gebildet.

Bei der in Fig. 2F gezeigten Variante, bei der die flexiblen und/oder elastischen Abschnitte 32 durch unterbrochene Linien nur angedeutet sind, können diese Abschnitte 32 bspw. durch in das textile Gewebe oder Gestricke des Shirts 22 bspw. durch elastische Bänder oder Tapes 38 gebildet sein, die durch Hitzeeinwirkung in eine stoffschlüssige Verbindung mit dem textilen Material des Shirts 22 gebracht sind. Diese Tapes 38 können wahlweise an der dem Patienten 10 zugewandten Innenseite und/oder an der Außenseite des Shirts 22 angebracht bzw. eingearbeitet sein.

Bei der in Fig. 2E gezeigten Variante fehlen die Haftflächen 28 auf den Wirkflächen 24, da die Zugbänder 34 hierbei direkt auf die Wirkflächen 24 appliziert und dauerhaft dort fixiert sind, da eine Ablösung, eine Positionsveränderung und/oder ein Austausch der Zugbänder 34 nicht vorgesehen ist. Die Zugbänder 34 können mit ihren dem Shirt 22 zugewandten Flächen bspw. auf dem textilen Trägermaterial des Shirts 22 aufgeklebt oder durch Hitzeeinwirkung verschweißt werden. Die Zugbänder 34 können mit ihren dem Shirt 22 zugewandten Endabschnitten auch mit den Wirkflächen 24 vernäht werden.

Eine hier nicht gezeigte Variante kann zudem auf verstärkte Wirkflächen 24 verzichten und vorzugsweise etwas breitere Zugbänder 34 als in den Figuren 2B ff. gezeigt verwenden, die mit dem textilen Material des Shirts 22 verklebt, verschweißt oder vernäht sein können. Die von den Zugbändern 34 erzeugten Zugkräfte können ggf. auch ohne Unterstützung der beschriebenen Wirkflächen 24 in das textile Rumpfkorsett 20 eingebracht werden.

Eine weitere Variante eines erfindungsgemäßen textilen Rumpfkorsetts 20 ist in der schematisierten Rückansicht der Fig. 3 gezeigt, die wiederum einen aufrechtstehenden Patienten 10 verdeutlicht.

Auch bei dieser Variante kann das erfindungsgemäße textile Rumpfkorsett 20 vorzugsweise einem Oberbekleidungsstück ähneln und bspw. durch ein Bekleidungsstück ähnlich einem Pullover oder einem kurzärmeligen Shirt 22 gebildet sein, wie dies bereits oben unter Bezugnahme auf die Figuren 2A ff. erläutert wurde. Die in der Rückansicht erkennbaren mindestens zwei Wirkflächen 24, die vorzugsweise durch verstärkte textile Bereiche 26 gebildet sein können, sind hierbei etwas kleiner ausgebildet als bei den zuvor gezeigten Varianten.

Die verstärkten textilen Bereiche 26 oder Wirkflächen 24 sind auch in der Darstellung der Fig. 3 jeweils durch unterbrochene Linien angedeutet. Eine linke Wirkfläche 24 ist hierbei etwas größer und befindet sich oberhalb der Hüfte 14 auf der linken Körperhälfte des Patienten 10. Eine deutlich kleinere Wirkfläche 24 befindet sich ebenfalls oberhalb der Hüfte, jedoch auf der rechten Körperhälfte des Patienten 10. Die linken und rechten Wirkflächen sind jeweils am Rückenbereich als auch am hier nicht sichtbaren vorderen Körperbereich voneinander beabstandet. Die beiden Wirkflächen 24 sind jeweils durch verstärkte textile Bereiche 26 gebildet, die durch ihre Integration in das textile Material des Shirts 22 jeweils flächig an den gezeigten linken und rechten Rumpfbereichen des Patienten 10 auf- oder anliegen.

Auf die Darstellung von Haftflächen gemäß Figuren 2A bis 2D ist hier verzichtet. Solche Haftflächen, bspw. gebildet durch Klettflächen o. dgl., können jedoch in gleicher Weise vorhanden sein, wie es dort gezeigt ist. Die direkt mit dem textilen Material der verstärkten textilen Bereiche 26 der Wirkflächen 24 verbundenen oder mittels Haft- oder Klettflächen dort verankerten flexiblen elastischen Abschnitte 32 weisen jedoch eine andere Gestalt auf als bei der in den Figuren 2A ff. gezeigten Variante. Anstelle der dort eingesetzten einfachen elastischen Zugbänder 34 können gemäß Fig. 3 auch verzweigte elastische Zugbänder 40 eingesetzt werden, bei denen sich mindestens ein Abschnitt, der zwischen zwei Wirkflächen 24 verläuft und diese zugkraftübertragend verbindet, in einen weiteren Abschnitt verzweigt, dessen Ende entweder an anderer Stelle mit einer der beiden zugkraftübertragend verbundenen Wirkflächen 24 oder mit einer weiteren Wirkfläche (hier nicht gezeigt) verbunden und dort verankert ist.

Im gezeigten Ausführungsbeispiel der Fig. 3 ist das verzweigte elastische Zugband 40 mit einem Zweig an der kleineren rechten Wirkfläche 24 und mit seinen beiden anderen Zweigen oben und unten an der größeren linken Wirkfläche 24 verankert.

Wie dies schon zuvor betont wurde, können auch derartige verzweigte Zugbänder 40 vielfältige Gestaltungen aufweisen und durch die Stärke, die Länge, die Rückstellkräfte und die Konturen der Zugbänder 40 sowie deren Zweigabschnitte individuell an das Krankheitsbild und die besondere Situation des jeweiligen Patienten 10 angepasst werden. Es muss deshalb an dieser Stelle nicht betont werden, dass das gezeigte Ausführungsbeispiel nicht einschränkend zu verstehen ist, sondern lediglich die der Erfindung zugrunde liegenden Prinzipien der Korrektor von Fehlhaltungen des Körperrumpfes des Patienten 10 verdeutlichen soll.

Ähnlich der schon anhand der Fig. 2C verdeutlichten Variante können auch die Zweigabschnitte des in Fig. 3 beispielhaft gezeigten verzweigten Zugbandes 40 unterschiedlich breit ausgeführt sein, wobei die Zweigabschnitte nahezu beliebig schräg oder diagonal am Körper verlaufen können. Auf diese Weise kann auch ein solches einfach oder wahlweise auch mehrfach verzweigtes Zugband 40 die hier angestrebte Aufgabe erfüllen, mittels der Auswahl der passend dimensionierten einfachen oder verzweigten Zugbänder 34 und/oder 40, die jeweils unterschiedliche Elastizitäten aufweisen und damit veränderliche Zug- und Rückstellkräfte zwischen den miteinander verbundenen Wirkflächen 24 erzeugen, dem textilen Rumpfkorsett 20 die für den jeweiligen Patienten 10 passenden Stützkräfte aufzuprägen, die es dem Patienten erleichtern, eine verbesserte aufrechte Körperhaltung einzunehmen, ohne dass er hierfür zwingend ein steifes Halbschalenkorsett tragen müsste.

Unterschiedliche verzweigte Zugbänder 40 mit jeweils unterschiedlichen Stärken und von ihnen erzeugten Zugkräften können bspw. unterschiedliche Farben aufweisen und solchermaßen kodiert sein, um bei der Anpassung des textilen Rumpfkorsetts 20 an den Träger eine Auswahl aus einer begrenzten Palette von verzweigten Zugbändern 40 treffen zu können.

Auch wenn die Fig. 3 nur jeweils die Rückseite des durch das Shirt 22 gebildete textilen Rumpfkorsetts 20 zeigen, ist es wichtig zu betonen, dass sich an der Vorderseite des Rumpfkorsetts 20 entweder eine ähnliche Konfiguration mit mindestens einem Zugband 34 (gemäß Fig. 2A bis Fig. 2F), einem einfach oder mehrfach verzweigten Zugband 40 oder einem anderen geeigneten flexiblen und/oder elastischen Abschnitt zur Verbindung der auch an der Vorderseite voneinander beabstandeten Wirkflächen 24 befindet, oder dass die rückseitig voneinander beabstandeten Wirkflächen 24 an der Vorderseite des Shirts 22 ineinander übergehen oder miteinander verbunden sind. Je nach individueller Ausprägung des zu behandelnden Krankheitsbildes des Patienten 10 kann die eine oder andere Variante sinnvoll eingesetzt werden. Während die erstgenannte Variante mit den vorderseitig angeordneten lösbaren und austauschbaren Zugbändern 34 oder dem mindestens einen vorderseitig angeordneten verzweigten Zugband 40 umfangreichere Anpassungs- und Einstellungsmöglichkeiten bieten, kann die alternative Variante ohne Zugbänder und mit stattdessen vorderseitig verbundenen Wirkflächen für manche schwächere Ausprägungen von Wirbelsäulenfehlstellungen eine vereinfachte Anpassung und günstigere Fertigung des textilen Rumpfkorsetts ermöglichen.

Zur Herstellung und zu abgewandelten Varianten der in Fig. 3 gezeigten Variante des textilen Rumpfkorsetts 20 sei auf die Figuren 2D bis 2F verwiesen, die auch bei der hier diskutierten Variante mit dem verzweigten Zugband 40 oder mehreren verzweigten Zugbändern 40 gleichermaßen zur Anwendung kommen können. So können die flexiblen und/oder elastischen Abschnitte 32 entweder gemäß Fig. 2E und Fig. 3 durch fest verankerte, außen auf dem Shirt 22 aufgebrachte und dessen Wirkflächen 24 unter Herstellung von definierten Zugkräften zwischen diesen verbindende verzweigte Zugbänder 40 oder durch in das textile Material des Shirts 22 eingearbeitete flexible und/oder elastische Abschnitte 32 gemäß Fig. 2F gebildet sein.

Auch für die Fig. 3 gilt das zuvor gesagte, nach dem eine hier nicht gezeigte Variante ggf. auf verstärkte Wirkflächen 24 verzichten und vorzugsweise etwas breitere verzweigte Zugbänder 40 als in Fig. 3 gezeigt aufweisen kann, wobei diese Zugbänder 40 bspw. mit dem textilen Material des Shirts 22 verklebt, verschweißt oder vernäht sein können. Die von den Zugbändern 40 erzeugten Zugkräfte können ggf. auch ohne Unterstützung der beschriebenen Wirkflächen 24 in das textile Rumpfkorsett 20 eingeleitet werden.

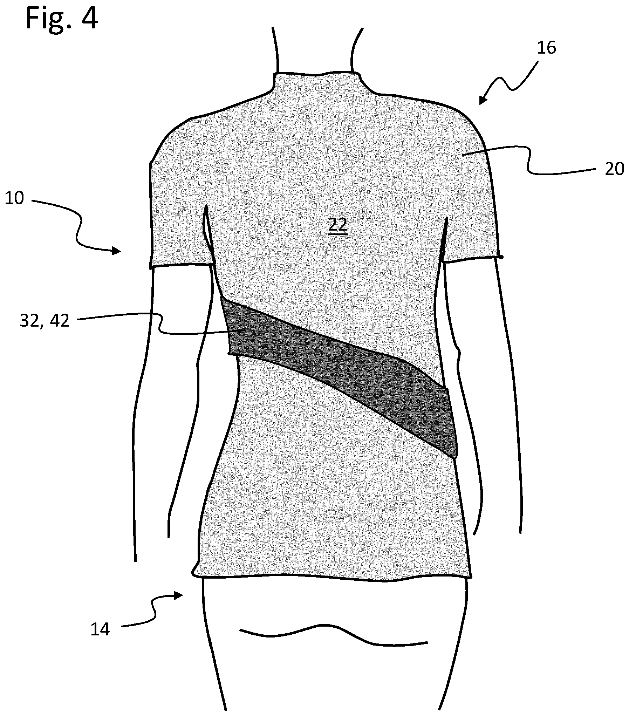

Eine weitere Variante eines erfindungsgemäßen textilen Rumpfkorsetts 20 ist in der schematisierten Rückansicht der Fig. 4 gezeigt, die wiederum einen aufrechtstehenden Patienten 10 verdeutlicht.

Auch bei dieser Variante kann das erfindungsgemäße textile Rumpfkorsett 20 vorzugsweise einem Oberbekleidungsstück ähneln und bspw. durch ein Bekleidungsstück ähnlich einem Pullover oder einem kurzärmeligen Shirt 22 gebildet sein, wie dies bereits oben unter Bezugnahme auf die Figuren 2A ff. sowie der Fig. 3 erläutert wurde. Wirkflächen sind in dieser Rückansicht nicht erkennbar; solche Wirkflächen, wie sie zuvor beschrieben wurden, können sich allerdings an der hier nicht sichtbaren Frontseite des textilen Rumpfkorsetts befinden, typischerweise gebildet jeweils durch verstärkte textile Bereiche.

Sofern dort solche durch verstärkte textile Bereiche gebildeten Wirkflächen vorhanden sind, können diese insbesondere durch ihre Integration in das textile Material des Shirts 22 jeweils flächig an den jeweiligen Rumpfbereichen des Patienten 10 auf- oder anliegen. Wahlweise kann auch die anhand der Fig. 4 erläuterte Ausführungsvariante des textilen Rumpfkorsetts 20 Haftflächen, bspw. gebildet durch Klettflächen o. dgl., aufweisen, um mindestens einen flexiblen Abschnitt dort zu verankern.

Das in Fig. 4 gezeigte textile Rumpfkorsett 20 ist im Wesentlichen durch ein breiteres Zugband 42 charakterisiert, das den flexiblen Abschnitt 32 bildet. Das breitere Zugband 42 verläuft quer über den Rücken des Patienten 10 und ist mit seinen beiden Enden an der Frontseite des Shirts 22 befestigt, wahlweise über hier nicht gezeigte Haftflächen, durch eine komplette oder abschnittsweise verlaufende Verankerung und/oder Integration in das textile Material des Shirts 22. Denkbar ist auch eine Variante, bei der die normalerweise voneinander beabstandeten Enden des breiteren Zugbandes 42 aneinander stoßen und/oder überlappen oder miteinander verbunden sind, so dass eine geschlossene Schlaufe gebildet ist, die horizontal oder abschnittsweise diagonal um den Körper des Patienten 10 herum verläuft.

Wie dies schon zuvor anhand der oben erläuterten Ausführungsvarianten betont wurde, kann auch ein solches breiteres Zugband 42 vielfältige Gestaltungen aufweisen und durch die Stärke, die Länge, die Rückstellkräfte und die Konturen des Zugbandes 42 sowie dessen Verlauf über der Oberfläche des Shirts 22 individuell an das Krankheitsbild und die besondere Situation des jeweiligen Patienten 10 angepasst werden. Es muss deshalb an dieser Stelle nicht betont werden, dass das in Fig. 4 gezeigte Ausführungsbeispiel nicht einschränkend zu verstehen ist, sondern lediglich die der Erfindung zugrunde liegenden Prinzipien der Korrektor von Fehlhaltungen des Körperrumpfes des Patienten 10 verdeutlichen soll.

Auch das in Fig. 4 beispielhaft gezeigte breitere Zugband 42 kann unterschiedlich breit ausgeführt sein, wobei der Verlauf nahezu beliebig schräg oder diagonal am Körper entlangführen kann. Auf diese Weise kann auch ein solches breiteres Zugband 42, das ggf. auch einfach oder wahlweise auch mehrfach verzweigt sein kann, die hier angestrebte Aufgabe erfüllen, mittels der Auswahl des passend dimensionierten einfachen oder verzweigten breiteren Zugbandes 42, das angepasste und/oder unterschiedliche Elastizitäten aufweisen und damit veränderliche Zug- und Rückstellkräfte zwischen den miteinander verbundenen Wirkflächen 24 erzeugen kann, dem textilen Rumpfkorsett 20 die für den jeweiligen Patienten 10 passenden Stützkräfte aufzuprägen, die es dem Patienten erleichtern, eine verbesserte aufrechte Körperhaltung einzunehmen, ohne dass er hierfür zwingend ein steifes Halbschalenkorsett tragen müsste.

Zur Herstellung und zu abgewandelten Varianten der in Fig. 4 gezeigten Variante des textilen Rumpfkorsetts 20 sei an dieser Stelle wiederum auf die Figuren 2D bis 2F verwiesen, die auch bei der hier diskutierten Variante mit dem breiteren Zugband 42 gleichermaßen zur Anwendung kommen können. So kann der flexible und/oder elastische Abschnitt 32 entweder gemäß Fig. 2E und Fig. 3 durch ein fest verankertes, außen auf dem Shirt 22 aufgebrachtes und dessen Wirkflächen 24 unter Herstellung von definierten Zugkräften zwischen diesen verbindendes breiteres Zugband 42 oder durch einen in das textile Material des Shirts 22 eingearbeiteten flexiblen und/oder elastischen Abschnitt 32 gemäß Fig. 2F gebildet sein.

Auch für die Fig. 4 gilt das zuvor gesagte, nach dem das breitere Zugband 42 bspw. mit dem textilen Material des Shirts 22 verklebt, verschweißt oder vernäht sein kann. Die vom Zugband 42 erzeugten Zugkräfte können ggf. auch ohne Unterstützung der beschriebenen Wirkflächen 24 in das textile Rumpfkorsett 20 eingeleitet werden.

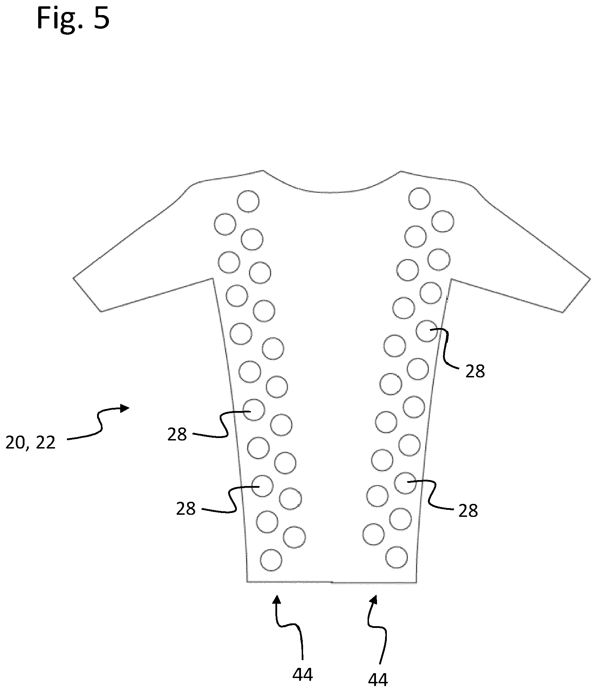

Die schematische Darstellung der Fig. 5 zeigt schließlich eine Rückseite eines Oberbekleidungsstückes 22, das mit zahlreichen Verankerungsflächen 28 zum lösbaren Applizieren von elastischen Zugelementen 32 oder 34 (vgl. hierzu die Figuren 2B ff.) ausgestattet ist. Die kreisrunden Verankerungsflächen 28, welche die Abschnitte zur Verankerung von dort haftenden Endbereichen der Zugelemente 32 oder 34 (hier nicht gezeigt) bilden, sind jeweils beabstandet voneinander angeordnet, aber in einer Weise gruppiert, dass streifenartige Areale 44 von sich in vertikaler Richtung aneinanderreihenden Abschnitten oder Verankerungsflächen 28 ergeben.

Wie es die Fig. 5 beispielhaft verdeutlicht, sind die streifenartigen Areale 44 so gestaltet, dass jedes dieser Areale 44 links und rechts entlang der linken und rechten Rückseite des Oberbekleidungsstückes 22 erstrecken, und zwar von dessen unterer Kante (Hüftbereich) bis zum Schulterbereich (ggf. bis zum oberen Ausschnitt), wobei die Areale 44 von unten nach oben leicht V-förmig auseinanderstreben.

Im gezeigten Ausführungsbeispiel umfasst jedes der beiden streifenartigen Areale 44 insgesamt zwanzig kreisrunde Abschnitte oder Verankerungsflächen 28, wobei diese paarweise versetzt zueinander angeordnet sind.

Zur Herstellung eines therapeutisch wirkenden Oberbekleidungsstückes 22 können die Abschnitte oder Verankerungsflächen 28 jeweils zum Applizieren geeigneter Zugelemente 34 an geeigneten Stellen genutzt werden, was hier jedoch nicht näher gezeigt ist.

Außerdem kann nach dem Aufsetzen einiger Zugelemente 34 an sinnvoll erscheinenden Positionen eine optische Vermessung des mit dem Oberbekleidungsstück 22 bekleideten Trägers oder Patienten 10 und seiner Oberkörperposition sowie dessen Stellung und/oder Position vorgenommen werden. Bei der Vermessung des mit dem Oberbekleidungsstück 22 bekleideten Trägers variiert seine Oberkörperposition sowie seine Stellung und/oder Position unter der Wirkung mindestens eines zwischen zwei voneinander entfernten Abschnitten platzierten Zugelementes 34 und damit unter der Wirkung der von dem mindestens einen Zugelement 34 zwischen den voneinander entfernten Verankerungsflächen 28 ausgeübten Zugkräften.

Die optische Vermessung erfolgt sinnvollerweise unter Verwendung der in Fig. 2D gezeigten optischen Referenzpunkte 36, die in Fig. 5 nicht gezeigt, vorzugsweise jedoch an mehreren geeigneten Stellen vorhanden sind. Mittels der optischen Vermessung unter Zuhilfenahme mehrerer optischer Referenzpunkte 36 kann die Wirksamkeit jeder Maßnahme, d.h. die Wirksamkeit jeder Applikation einzelner Zugelemente 34 überprüft und verifiziert werden. Wenn die optische Vermessung nach jeder Variation der aufgebrachten Zugelemente 34 unter Erfassung der optisch erkennbaren Referenzpunkte 36 am Oberbekleidungsstück 22 erfolgt, kann das gewünschte hohe Maß an Reproduzierbarkeit bei jedem Messvorganges gewährleisten werden.

Bei dem Verfahren können in beliebiger Abfolge mehrere optische Vermessungen nach Anpassung und/oder Austausch der Zugelemente 34 und/oder unter Variation der Positionen mindestens eines Zugelementes 34 oder mehrerer Zugelemente 34 durchgeführt werden, so dass eine optimierte Wirkung erzielt und gleichzeitig überprüft werden kann.

Auf diese Weise kann etwa ein mit der Anpassung eines durch das gezeigte Oberbekleidungsstück 22 gebildete textile Rumpfkorsett befasster Physiotherapeut oder Orthopäde die passend ausgesuchten Zugelemente 34 oder elastischen Zugbänder 34 auf den ihm sinnvoll erscheinenden Verankerungsflächen 28 positionieren und anschließend die am Patienten wirksam gewordene Korrektur seiner Körperhaltung kontrollieren.

Die Vorderseite des Oberbekleidungsstückes 22 kann in gleicher Weise ausgestaltet sein wie die in Fig. 5 gezeigte Rückseite, so dass dort ebenfalls Zugelemente 34 an unterschiedlichen Positionen verankert und auch wieder umpositioniert werden können, sobald sich dies als sinnvoll oder notwendig erweist.

Nach einer optischen Vermessung eines solchermaßen mit mehreren Zugbändern 34 ausgestatteten Oberbekleidungsstückes 22 können die gewonnenen Messdaten wahlweise zur Anfertigung eines Shirts 22 mit eingearbeiteten oder integrierten Tapes 38 gemäß Fig. 2F genutzt werden.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

- 10: Patient
- 12: Korsett, Chêneau-Korsett
- 14: Hüfte
- 16: Schulter, Schulterbereich
- 20: Rumpfkorsett, textiles Rumpfkorsett
- 22: Shirt, Oberbekleidung, textiles Shirt, Oberbekleidungsstück
- 24: Wirkfläche, Abschnitt
- 26: verstärkter textiler Bereich
- 28: Haftfläche, Verankerungsfläche
- 30: Klettfläche
- 32: flexibler Abschnitt, elastischer Abschnitt, flexibler und/oder elastischer Abschnitt
- 34: Zugband, elastisches Zugband, Zugelement
- 36: Referenzpunkt, optischer Referenzpunkt
- 38: Tape
- 40: verzweigtes Zugband, verzweigtes elastisches Zugband
- 42: breites Zugband
- 44: Areal, streifenartiges Areal

## Patentansprüche

1. Oberbekleidungsstück (22) zur äußeren Einwirkung auf einen Oberkörper eines Trägers oder Patienten (10) mittels Stütz- und/oder Zugkräften, insbesondere zur Abstützung und/oder zur therapeutischen Behandlung von Wirbelsäulenstellungen, die von einer aufrechten Normalstellung abweichen, etwa im Zusammenhang mit dem Krankheitsbild einer Skoliose,
- wobei sich zwischen mindestens zwei voneinander entfernten Abschnitten (24, 28) des Oberbekleidungsstückes (22) jeweils mindestens ein Zugelement (32, 34) befindet, das die mindestens zwei voneinander beabstandete Abschnitte (24, 28) verbindet und Zugkräfte zwischen den mindestens zwei Abschnitten (24, 28) erzeugt,
- wobei das mindestens eine Zugelement (32, 34) über elastische Eigenschaften verfügt, die sich von den elastischen Eigenschaften des Oberbekleidungsstückes (22) dahingehend unterscheiden, dass die elastischen Rückstellkräfte des mindestens einen Zugelementes (32, 34) stärker sind als diejenigen Bereiche des Oberbekleidungsstückes (22), die sich zwischen den mindestens zwei Abschnitten (24, 28) befinden.

2. Oberbekleidungsstück nach Anspruch 1, bei dem das mindestens eine Zugelement (32, 34) durch ein elastisches Band gebildet ist, dessen beide Enden jeweils an den voneinander beabstandeten Abschnitten (24, 28) verankert sind.

3. Oberbekleidungsstück nach Anspruch 1 oder 2, an dessen Außenseite sich mehrere voneinander entfernte Abschnitte (24, 28) befinden.

4. Oberbekleidungsstück nach einem der Ansprüche 1 bis 3, bei dem die Abschnitte (24, 28) jeweils gruppiert sind, wobei diese Gruppen mit jeweils mehreren voneinander beabstanden Abschnitten (24, 28) jeweils größere Bereiche bilden.

5. Oberbekleidungsstück nach Anspruch 4, bei dem die Bereiche mit den mehreren Abschnitten (24, 28) als streifenartige Areale (44) ausgebildet sind, die sich jeweils entlang einer vertikalen Richtung der getragenen Oberbekleidung (22) erstrecken.

6. Oberbekleidungsstück nach Anspruch 4 oder 5, bei dem die Bereiche oder streifenartigen Areale (44) sich rückseitig und/oder vorderseitig jeweils entlang der linken und rechten Seite des Oberbekleidungsstückes (22) erstrecken.

7. Oberbekleidungsstück nach einem der Ansprüche 1 bis 6, bei dem die Abschnitte (24, 28) jeweils als erste Gegenflächen eines Verschlussmittels, insbesondere eines Klettverschlusses ausgebildet sind, und bei dem die Zugmittel (32, 34) jeweils mit zweiten Gegenflächen des Verschlussmittels ausgestattet sind.

8. Verfahren zur Anpassung und/oder Variation von auf einen Oberkörper eines Trägers oder Patienten (10) einwirkenden Stütz- und/oder Zugkräften mittels eines Oberbekleidungsstückes (22), welches insbesondere der Abstützung und/oder zur therapeutischen Behandlung von Wirbelsäulenstellungen, die von einer aufrechten Normalstellung abweichen, dienen kann,
- wobei sich zwischen mindestens zwei voneinander entfernten Abschnitten (24, 28) des Oberbekleidungsstückes (22) jeweils mindestens ein zumindest einseitig von einem der mindestens zwei Abschnitte (24, 28) lösbares Zugelement (32, 34) befindet,
- wobei das mindestens eine Zugelement (32, 34) in einem mit den beiden entfernten Abschnitten (24, 28) jeweils verbundenen Zustand die mindestens zwei voneinander beabstandeten Abschnitte (24, 28) verbindet und Zugkräfte zwischen den mindestens zwei Abschnitten (24, 28) erzeugt,
- wobei das mindestens eine Zugelement (32, 34) über elastische Eigenschaften verfügt, die sich von den elastischen Eigenschaften des Oberbekleidungsstückes (22) dahingehend unterscheiden, dass die elastischen Rückstellkräfte des mindestens einen Zugelementes (32, 34) höher sind als diejenigen Bereiche des Oberbekleidungsstückes (22), die sich zwischen den mindestens zwei Abschnitten (24, 28) befinden,
- und wobei die auf das Oberbekleidungsstück (22) und damit auf den Oberkörper des Trägers oder Patienten (10) einwirkenden Stütz- und/oder Zugkräfte durch Variation einer Positionierung des mindestens einen Zugelementes (32, 34) zwischen den mindestens zwei Abschnitten (24, 28) und/oder durch dessen zumindest einseitiges Lösen von einem der Abschnitte (24, 28) verändert werden können.

9. Verfahren nach Anspruch 8, bei dem die Zugelemente (32, 34) umgesetzt und unter Variation ihrer jeweils wirksamen Länge anders auf den Abschnitten (24, 28) aufgesetzt werden können.

10. Verfahren nach Anspruch 8 oder 9, bei dem die Zugelemente (32, 34) gegen Zugelemente (32, 34) anderer Länge und/oder anderer elastischer Eigenschaften ausgetauscht werden können.

11. Verfahren nach einem der Ansprüche 8 bis 10, bei dem eine optische Vermessung des mit dem Oberbekleidungsstück (22) bekleideten Trägers und seiner Oberkörperposition sowie dessen Stellung und/oder Position als Auswirkung mindestens eines zwischen zwei voneinander entfernten Abschnitten (24, 28) platzierten Zugelementes (32, 34) und damit als Auswirkung der von dem mindestens einen Zugelement (32, 34) zwischen den voneinander entfernten Abschnitten (24, 28) ausgeübten Zugkräften erfolgt.

12. Verfahren nach Anspruch 11, bei dem die optische Vermessung unter Erfassung von optisch erkennbaren Referenzpunkten (36) am Oberbekleidungsstück (22) erfolgt.

13. Verfahren nach Anspruch 11 oder 12, bei dem die optisch erkennbaren Referenzpunkte (36) jeweils den Abschnitten (24, 28 zugeordnet sind.

14. Verfahren nach einem der Ansprüche 11 bis 13, bei dem mehrere optische Vermessungen nach Anpassung und/oder Austausch der Zugelemente (32, 34) und/oder unter Variation der Positionen mindestens eines Zugelementes (32, 34) oder mehrerer Zugelemente (32, 34) durchgeführt werden.
